# EUROPEAN PATENT SPECIFICATION

(11) **EP 0 411 932 B1**
(45) Date of publication and mention of the grant of the patent: **21.02.1996**
(21) Application number: 90308483.8
(22) Date of filing: 01.08.1990
(51) Int. Cl.: G01N 1/08

(54) **Sampling probe**
Probensonde
Sonde d'échantillonnage

(30) Priority: 02.08.1989 GB 8917645; 06.04.1990 GB 9007893
(43) Date of publication of application: 06.02.1991
(73) Proprietor: Samplex Ltd, North Walsham, Norfolk NR28 OBD (GB)
(72) Inventor: Catchpole, David, North Walsham, Norfolk NR28 OBD (GB)
(74) Representative: Maury, Richard Philip

(56) References cited:
- EP-A- 0 251 411
- FR-A- 2 542 086
- US-A- 3 580 084
- US-A- 4 088 025
- US-A- 4 283 946
- US-A- 4 616 515

## Description

This invention relates to a sampling probe for extracting representative samples from bulk material, and to a method of doing so, and is especially useful in the sampling of powdered solid or granular materials, for example grain or flour, which may comprise a mixture of particles of different sizes including fine dust. The invention is particularly useful in the extraction of a sample obtained uniformly from each part of a vertical column of the bulk material.

Sampling probes in the form of spears, or triers as they are also known, are conventionally used in food grain sampling, where the careful monitoring of the condition of the grain is controlled by British Standards, and of seeds by Seed Regulations. It is important to obtain truly representative samples of the bulk material, yet this is difficult when the material comprises a mixture of different sized components ranging from dust to complete grains. Using a typical automatic vacuum-driven sampling spear consisting of a pointed tube with an aperture in the side wall near its tip, the percentage of dust by weight in a sample was 21% when the deliberate spike in the bulk material was 16.7%; using a typical hand-held spear, the percentage dust from the same bulk material was 11.4%. This magnitude of error is unacceptable, and the purpose of the invention, in one aspect, is to provide a sampling probe which takes a more representative sample.

The invention, in its first aspect, provides a pneumatic sampling probe according to Claim 1.

One example of such a probe has been found to take a much more representative sample of the bulk material referred to above, in which the percentage by weight of dust was 16.9%, i.e. the composition of components of different particle size in the samples it takes is accurate to a little over one percent. It is believed that this improvement is due to the manner in which the probe admits the sample; it allows the sample to enter through the aperture into the intermediate store, assisted by a small pressure difference which is limited by the presence of the intermediate store, and neither under gravity alone nor by direct suction. Thus there is no influence tending to select one particle size in favour of another: the aperture is preferably large in relation to the particle size for which the sampling probe is intended. Although the pressure is substantially lower at the outlet, the intermediate store of sample material is capable in use of acting as a buffer, so that the pressure experienced by the material as it is sucked from the intermediate store decreases smoothly from atmospheric pressure.

The invention, in a second aspect, also provides a method of withdrawing a representative sample from bulk material using a sampling probe according to the first aspect of the invention described above, as defined in Claim 4.

From a third aspect, the invention provides a method as defined in Claim 6 of withdrawing a representative sample from bulk material.

Conventional hand probes for grain sampling, such as those described in U.S. Patent No. 2896444, comprise a spear-like body which is plunged into the bulk material, and a series of spaced apertures for admitting samples into an inner collection chamber. Automated probes are also known, for example that disclosed in French Patent No. 2542086, in which the sampled material is extracted from the probe by suction. Neither form of sampling apparatus takes a truly representative sample, and it is believed that this is at least partly because of the way in which the particles fall into the chamber; there is inherently a bias towards the collection of either larger or smaller particles. In the case of the hand-held probes of US-2896444, for example, it is believed that the particles fall in an irregular path inclined at about 45 degrees to the vertical probe axis, before they reach the apertures, and that this results in a higher grain-to-dust ratio in the sample than in the bulk material.

Moreover, conventional probes are suited only to specific types of bulk material, so that a range of probes is required.

Using the invention, the sample can be as representative as possible, and the probe is capable of use with a wide range of different bulk materials and mixtures.

With each aspect of the invention as described above, the sampling system may be automatic, in which the motion of the sampling device (probe) and the extraction of the sample for analysis are performed under automatic control, or it may be manual.

Sampling systems embodying the invention will now be described, by way of example, with reference to the accompanying drawings, in which:-
Figure 1 is a side elevation and Figure 2 is a front elevation of a sampling spear;
Figures 3 and 4 are front and side elevations, to an enlarged scale, of the lower portion of the spear of Figures 1 and 2, with additional apparatus and with a differently-shaped aperture;
Figure 5 is a diagrammatic elevation of a pressure control device.

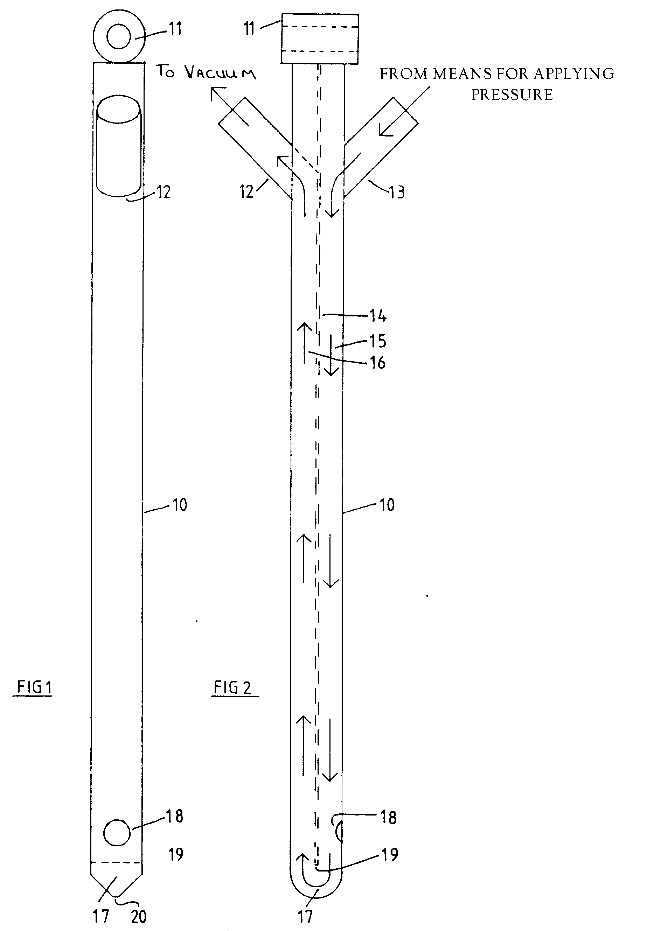

The sampling spear of Figures 1 and 2 comprises a steel wall 10 about 2m long closed at both ends and terminating at its upper end at a bush 11 for connection to an arm of an automatic sampling station, or a lever for manual insertion. Its lower end is formed into a pointed tip 20 to ease its insertion into bulk material such as grain.

The spear is divided internally down a vertical cross section by a steel baffle plate 14 which extends from the top end 11 down to near the tip 20, separated from the tip by a distance of about half the width of the spear. This defines a U-shaped channel of generally uniform cross-section comprising upstream 15 and downstream 16 sections joined by a bent portion 17. An air inlet tube 13 is connected to the upstream section 15 near the top, and an outlet tube 12 is connected to the downstream section 16 at the same point near the top.

An aperture 18 which may be of fixed size but is preferably of a size variable using an adjustable plate or otherwise, is formed in the side wall 10 upstream of the tip 20, upstream of the end of the baffle plate 19 by a distance of about the width of the channel. The aperture has an area of the same order as the cross-sectional area of the channel at that point. The portion of the channel beneath the aperture 18 and immediately beneath the end 10 of the baffle plate, i.e. the bent portion of the channel, constitutes an intermediate store for the material entering the aperture 18 when the spear is generally upright as shown.

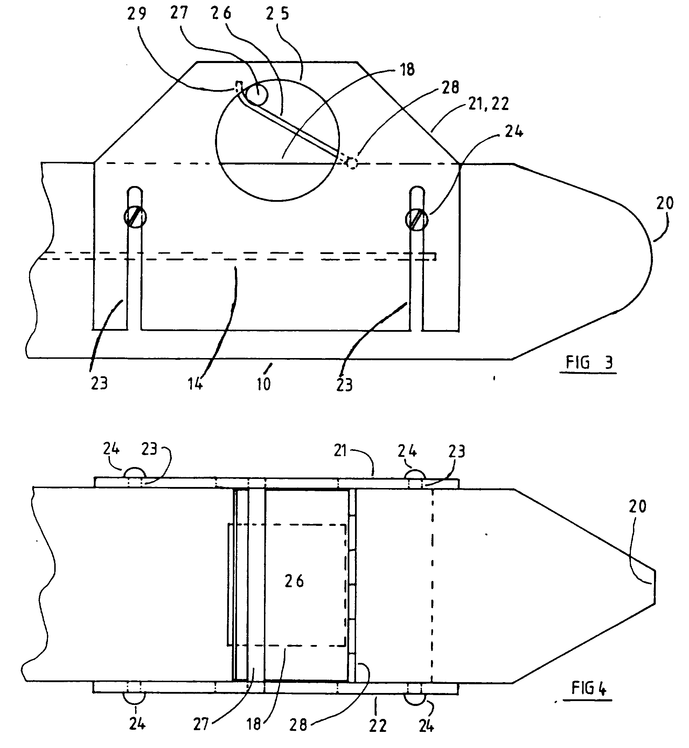

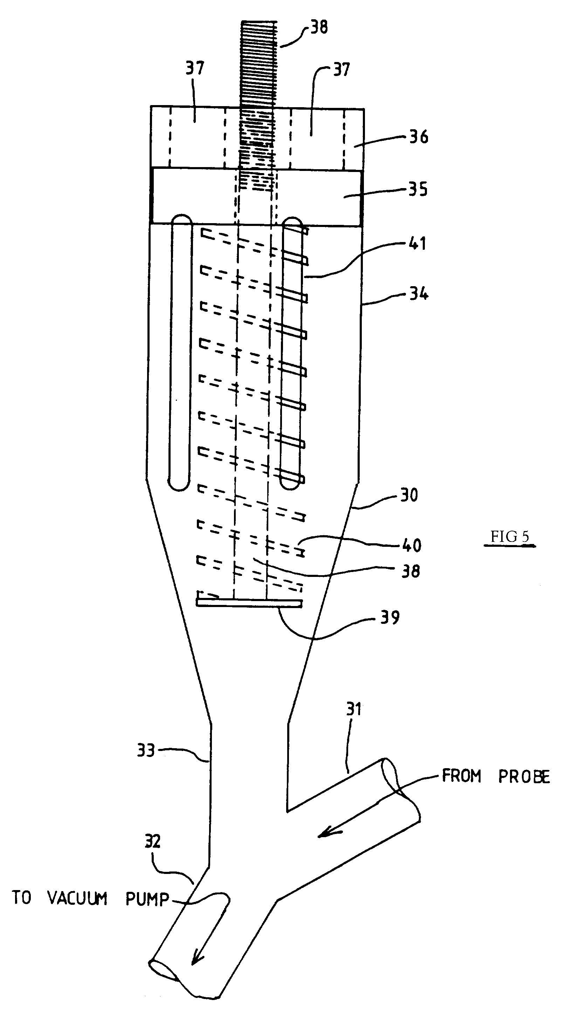

The accuracy of sample collection may be further enhanced, especially when the bulk is a flowable material, by the addition of a flap 26 on side wall 10, as shown in figures 3 and 4, which is closed on descent through the bulk, but opens upon ascent to scoop material into the channel.

In the modified spear shown in figures 3 and 4, the flap 26 is hinged along its lower edge 28 to the channel 10 outer wall immediately below the aperture 18, which in this example is rectangular (optionally adjustable by a plate). The flap has an outwardly curved lip 29 which reacts against the bulk material to move the flap outwardly or inwardly depending on whether the spear is ascending or descending. Outward movement of the flap is limited by a bar 27, attached to the flap and projecting therefrom at each side, abutting against a pair of stop plates 21, 22 having circular apertures 25. The transverse position of the stop plates 21, 22 is adjustable by virtue of slots 23 whose edges are clamped by bolts 24, so that the bar 27 abuts a corresponding part of the inner edges of the circular apertures 25, determining the maximum angle of opening of the flap 26.

The outlet tube 12 is connected to a conventional vacuum pump and grain sampling store. The air inlet 13 is connected to a means for applying an increased pressure to match the bulk material pressure at the point of its entry through aperture 18.

In use, the spear is pushed vertically downwards into the bulk grain, for example in a truck, until a major part is buried in the grain, ensuring that no grain enters the air inlet tube 13. Grain enters the channel through the aperture 18 due to the weight of the bulk material and the small pressure difference between the bulk and the inside of the channel, and falls into the intermediate store 17.

At a predetermined instant, the vacuum pump is activated, causing a pressure drop of approximately one atmosphere to develop across the intermediate store 17. Grain mixed with air is sucked up the downstream portion 16 of the channel, and through the outlet 12 to the external sample store.

In one method, without the flap, the sample is withdrawn as the spear descends, sampling then terminating when it reaches full depth; alternatively, the sample is withdrawn as it ascends, especially if the flap 26 is fitted. For less flowable materials, the sample is withdrawn on descent and ascent. Another method is for a sample to be withdrawn at a selected level which may be the deepest level. The sample is stored automatically; the spear is withdrawn, then moved transversely to a different sampling point, and then caused to sample again in the same way.

It will be apparent that the configuration of the spear may be varied from that shown; it may have a tubular section with a diametric baffle plate, for example, or it may comprise coaxial annular channel sections. The aperture may have a different shape, or there may be more than one aperture; there may be a projection inwardly or, as with flap 26, outwardly from the aperture.

To assist in the maintenance of a low pressure difference between inside and outside of the channel whilst in the bulk, holes may be provided in the baffle plate 14, which may vary in size.

Further, it is envisaged that the spear could be used for sampling damp or especially dense granular materials, or even liquids, as the vacuum pump maintains a steady flow along the channel.

Although in this example the spear has one channel only, the invention could be embodied in a more complex arrangement of plural channels, with plural inlet apertures communicating via respective intermediate stores in the tip region with one or more outlets.

The probe in this example is spear-shaped with a pointed tip, but other shapes appropriate for sampling the material concerned could be adopted.

As shown in Figure 9, the sample channel 31,32 from the sampling probe to the vacuum pump and sampling analysis store has a T-junction with an air inlet pipe 33. The device 30 has an air outlet connected to the pipe 33, and comprises an air chamber with frusto-conical and cylindircal 34 walls, the cylindrical wall 34 closed partially by an end plug 36 with apertures 37. A closure piston 35 slides sealingly against the wall 34 along a rod 38 whose screw-threaded end is engaged in the end plug 36. The piston is biased into abutment with the end plug by means of a coil spring 40 over the rod 38, retained thereon by an end stop plate 39, whose spring force is adjustable by the screw-threading on the rod 38.

The cylindrical wall 34 has four paraxial slots 41 open to the atmosphere, which are progressively obstructed by the piston 35. As the piston 35 moves towards the outlet 33 against the spring bias, under the atmospheric pressure on its outer surface, the portion of each aperture 41 seen by the air chamber progressively decreases, thus increasing progressively the resistance to the flow of atmospheric air into the chamber and thus into the sampling channel 31,32. In this way, the device responds automatically to the amount of suction in the channel 31,32 to vary that suction by controlling air flow. If it senses low suction, i.e. a pressure close to atmospheric, it will allow a substantial air flow; the aperture areas are of such a size, and the spring force is such, that a slight increase in suction will cause a slight movement of the piston. For this to occur, the minimum air flow resistance of the device, with the air chamber at its maximum size, needs to be comparable with the air flow resistance of the sampling device when empty.

In this way, any obstruction in the sampling device causes greater suction to be developed there, facilitating its clearance, but in the normal operation of the system the suction at the sampling device is just sufficient to convey the sample through the sampling channel. This feedback control helps to ensure that, where the sampling device has an aperture for receiving the sample from bulk material in which the device is immersed, the pressure difference across that aperture is kept very low, which as indicated above, provides for a representative sample.

## Claims

1. A pneumatic sampling probe for withdrawing a representative sample from bulk material into which the probe is inserted, comprising within an outer wall (10) a channel (15,16) for directing air along the probe to its tip (20) and thence back along the probe to an outlet (12) for connection to a vacuum pump and to a store for the sample, in which the wall (10) has an aperture (18) upstream of and close to the tip (20) exposing the channel to the exterior of the probe for the ingress of the sample, the bent portion of the channel between the aperture and the tip constituting an intermediate store (17) for the material to be sampled extending fully across the whole channel; characterised by air supply means connected to an upstream end (13) of the channel for supplying air at a pressure sufficient to match the pressure of the said bulk material, in use, at the said aperture (18), to facilitate the withdrawal of sampled material simultaneously with the ingress of the material under gravity through the aperture (18).

2. A probe according to Claim 1, in which the channel is U-shaped with its upstream and downstream sections divided by a baffle (19) extending longitudinally beyond the aperture towards the tip, so that the intermediate store (17) extends fully across the channel at its bent portion.

3. A probe according to Claim 1 or 2, in which the upstream section (15) of the channel extends substantially the full length of the probe.

4. A method of withdrawing a representative sample from bulk material using a sampling probe according to any of Claims 1 to 3, comprising inserting the probe downwardly tip first into the material, allowing the material to flow under gravity through the aperture (18) into the intermediate store (17), while simultaneously sucking air and the sampled material from the outlet (12) while allowing air from the air supply means into the upstream section (15) of the channel.

5. A method according to Claim 4, in which the air simultaneously admitted into the upstream section (15) of the channel is at a pressure which prevents the material entering the channel above the intermediate store (17) of material from experiencing any significant pressure change, but rather allows it to experience a smoothly decreasing pressure change as it passes downstream through the intermediate store (17) to the downstream section (16) of the channel.

6. A method of withdrawing a representative sample from bulk material using a sampling probe having a channel (15,16) for the passage of air and an aperture (18) exposing part of said channel to the exterior, comprising inserting the probe into the bulk material, and allowing the material to flow through said aperture (18) into an intermediate portion (17) to form an intermediate store across the whole channel, characterised by sucking air and the sampled material from the intermediate store (17) through the channel (16) downstream of the aperture, simultaneously with allowing the said material to flow under gravity through said aperture (18).

7. A pneumatic sampling system for fluid material such as granular material, comprising a vacuum pump connected through a sampling channel (31,32) to the outlet (12) of a probe according to Claim 1, 2 or 3, and, intermediate the probe and the pump, a pressure control device comprising: an air chamber defined by a wall (34) and having an air outlet (33) communicating with the sampling channel, the wall (34) having at least one opening (41) to the atmosphere, the opening being capable of progressive closure by a closure member (35) which is resiliently biased (40) towards a first position at which the opening is unobstructed, and the closure member being responsive to a difference in pressure between the atmosphere and the chamber interior to move against such resilient bias towards a second position at which the opening (41) is at least partially closed, causing a progressive closure of the opening, whereby the resistance to air flow from the atmosphere to the sampling channel increases progressively with a decreasing air pressure at the probe outlet (12).

## Patentansprüche

1. Pneumatische Probensonde zur Entnahme einer repräsentativen Probe aus Schüttgut, in welches die Sonde eingeführt ist, mit einem innerhalb einer Außenwand (10) angeordnetem Kanal (15, 16), um Luft entlang der Sonde zu deren Spitze (20) und von dort entlang der Sonde zurück zu einem Auslaß (12) zur Verbindung mit einer Vakuumpumpe und zu einem Lager für die Probe zu führen, wobei die Wand (10) eine Öffnung (18) stromaufwärts von und nahe der Spitze (20) aufweist, welche den Kanal zur Sonden-Umgebung für die Probennahme öffnet, wobei der gebogene Bereich des Kanals zwischen der öffnung und der Spitze ein Zwischenlager (17) für das zu entnehmende Material bildet, welches vollständig quer über den kompletten Kanal verläuft; gekennzeichnet durch eine Luftzufuhreinrichtung, welche mit einem stromaufwärts gelegenen Ende (13) des Kanals zum Zuführen von Druckluft verbunden ist, deren Druck ausreicht, um bei Verwendung mit dem Druck des Schüttguts an der Öffnung (18) übereinzustimmen, so daß die Entnahme des zu entnommenen Materials gleichzeitig mit dem Materialeintritt aufgrund der Schwerkraft durch die Öffnung (18) vereinfacht wird.

2. Sonde nach Anspruch 1, dadurch gekennzeichnet, daß der Kanal U-förmig ist, wobei dessen stromaufwärts und stromabwärts gelegenen Bereiche durch eine unterhalb der Öffnung zur Spitze längs verlaufende Trennung (19) unterteilt werden, so daß das Zwischenlager (17) vollständig über den Kanal an dessen gebogenem Bereich verläuft.

3. Sonde nach Anspruch 1 oder 2, dadurch gekennzeichnet, daß der stromaufwärts gelegene Bereich (15) des Kanals sich im wesentlichen über die komplette sondenlänge erstreckt.

4. Verfahren zur Entnahme einer repräsentativen Probe aus Schüttgut mittels einer Probensonde gemäß einem der Ansprüche 1 bis 3, mit den schritten:
Einführen der nach unten gerichteten Spitze der Sonde zuerst in das Schüttgut, um dem Schüttgut den Eintritt in das Zwischenlager (17) aufgrund von Schwerkraft durch die Öffnung (18) zu ermöglichen, während gleichzeitig Luft und das entnommene Material vom Auslaß (12) angesaugt wird, wobei Luft von der Luftzufuhreinrichtung in den stromaufwärts gelegenen Bereich (15) des Kanals einströmen kann.

5. Verfahren nach Anspruch 4, dadurch gekennzeichnet, daß die in den stromaufwärts liegenden Bereich (15) des Kanals gleichzeitig eingetretene Luft ein Druckniveau aufweist, welches ein Aufbringen einer wesentlichen Druckänderung auf das in den Kanal oberhalb des Zwischenlagers (17) des Schüttguts eintretenden Schüttguts verhindert, jedoch das Aufbringen einer gleichmäßig abnehmenden Druckänderung auf das Schüttgut ermöglicht, wenn es nach unten durch das Zwischenlager (17) zum stromabwärts liegenden Bereich (16) des Kanals strömt.

6. Verfahren zur Entnahme einer repräsentativen Probe aus Schüttgut mittels einer Probensonde, die einen Kanal (15, 16) für den Luftdurchlaß und eine Öffnung (18) aufweist, welche einen Kanalteil zur Umgebung öffnet, mit den Schritten: Einführen der Sonde in das Schüttgut und Einströmenlassen des Schüttguts durch die Öffnung (18) in einen Zwischenbereich (17), um ein Zwischenlager über den kompletten Kanal auszubilden, gekennzeichnet durch den Schritt: Ansaugen von Luft und von entnommenem Schüttgut aus den Zwischenlager (17) durch den Kanal (16) stromabwärts der Öffnung, gleichzeitig mit dem Hindurchströmenlassen des Schüttgutes mittels Schwerkraft durch die Öffnung (18).

7. Pneumatisches Probennahmesystem für Fluidmaterial, wie etwa Granulatmaterial, mit einer durch einen Probenkanal (31, 32) mit dem Auslaß (12) einer Sonde nach Anspruch 1, 2 oder 3 verbundenen Vakuumpumpe und einer zwischen der Sonde und der Pumpe angeordneten Drucksteuereinrichtung, mit: einer Luftkammer, welche durch eine Wand (34) ausgebildet ist und einen mit dem Probenkanal verbundenen Luftauslaß (33) aufweist, wobei die Wand (34) mindestens eine Öffnung (41) zur Atmosphäre aufweist, die Öffnung durch ein Verschlußelement (35) fortschreitend verschließbar ist, welches elastisch zu einer ersten Position vorgespannt ist (40), an welcher die Öffnung nicht blockiert ist und wobei das Verschlußelement auf eine Druckdifferenz zwischen der Atmosphäre und dem Kammerinnenraum anspricht, so daß sich das Verschlußelement gegen eine derartige elastische Vorspannung zu einer zweiten Position bewegt, an welcher die Öffnung (41) zumindest teilweise geschlossen ist, woraus ein fortschreitender Verschluß der Öffnung resultiert, wobei der Luftströmungswiderstand von der Atmosphäre zum Probenkanal fortschreitend mit einer Luftdruckabnahme am Sondenauslaß (12) zunimmt.

## Revendications

1. Sonde pneumatique d'échantillonnage pour retirer un échantillon représentatif d'un matériau en vrac dans lequel la sonde est introduite comprenant, dans une paroi extérieure (10) un canal (15, 16) pour diriger de l'air le long de l'échantillon vers sa pointe (20) et de là en arrière le long de la sonde vers une sortie (12) connectée à une pompe à vide et vers un stockage de l'échantillon, dans laquelle la paroi (10) a une ouverture (18) en amont et à proximité de la pointe (20), exposant le canal à l'extérieur de la sonde pour l'entrée de l'échantillon, la partie incurvée du canal entre l'ouverture et la pointe, constituant un stockage intermédiaire (17) pour le matériau à échantillonner, qui s'étend complètement en travers du canal entier, caractérisée par des moyens d'alimentation d'air connectés à une extrémité amont (13) du canal pour fournir de l'air à une pression suffisante pour atteindre la pression dudit matériau en vrac, présent à ladite ouverture (18), pour faciliter le retrait du matériau collecté simultanément à l'entrée du matériau sous l'effet de la gravité à travers l'ouverture (18).

2. Sonde selon la revendication 1, dans laquelle le canal a une forme de U, ses sections amont et aval étant divisées par une chicane (19) s'étendant longitudinalement au-delà de l'ouverture en direction de la pointe, de telle manière que le stockage intermédiaire (17) s'étende entièrement à travers le canal dans sa partie incurvée.

3. Sonde selon la revendication 1, dans laquelle la section amont (15) du canal s'étend sensiblement tout au long de la sonde.

4. Procédé pour retirer un échantillon représentatif d'un matériau en vrac, utilisant une sonde d'échantillonnage selon l'une quelconque des revendications 1 à 3, comprenant l'insertion de la sonde dans le matériau en y enfonçant la sonde vers le bas, la pointe la première, pour permettre au matériau de pénétrer sous l'effet de la gravité à travers l'ouverture (18) dans le stockage intermédiaire (17) en aspirant l'air et le matériau collecté par la sortie (12) tout en permettant l'arrivée d'air venant des moyens d'alimentation en air dans la section amont (15) du canal.

5. Procédé selon la revendication 4, dans lequel l'air simultanément admis dans la section amont (15) du canal est à une pression qui empêche le matériau de pénétrer dans le canal au-dessus du stockage intermédiaire (17) de matériau, en empêchant toute modification significative de pression, mais en permettant plutôt une diminution douce de pression lorsqu'il passe en aval à travers le stockage intermédiaire (17) vers la section aval (16) du canal.

6. Procédé pour retirer un échantillon représentatif d'un matériau en vrac, utilisant une sonde d'échantillonnage ayant un canal (15, 16) pour le passage de l'air et une ouverture (18) exposant une partie du canal vers l'extérieur, comprenant l'insertion de la sonde dans le matériau en vrac, et permettant au matériau de s'écouler à travers l'ouverture (18) dans une partie intermédiaire (17) pour former un stockage intermédiaire à travers tout le canal, caractérisé en ce que l'on aspire de l'air et le matériau collecté du stockage intermédiaire (17) à travers le canal (16) en aval de l'ouverture, tout en permettant simultanément audit matériau de s'écouler sous l'effet de la gravité à travers ladite ouverture (18).

7. Dispositif pneumatique d'échantillonnage pour un matériau fluide tel qu'un matériau granuleux, comportant une pompe à vide connectée à travers un canal d'échantillonnage (31, 32) à une sortie (12) d'une sonde, selon les revendications 1, 2 ou 3, et, entre la sonde et la pompe, un dispositif de contrôle de la pression comportant : une chambre à air définie par une paroi (34) et ayant une sortie d'air (33) communiquant avec le canal d'échantillonnage, la paroi (34) ayant au moins une ouverture (41) sur l'atmosphère, l'ouverture étant agencée pour être progressivement obturée par un organe d'obturation (35) qui est sollicitée élastiquement (40) vers une première position dans laquelle l'ouverture est fermée, et l'organe d'obturation étant activé en réponse à une différence de pression entre l'atmosphère et une chambre intérieure pour déplacer à nouveau la sollicitation élastique vers une seconde position dans laquelle l'ouverture (41) est au moins partiellement fermée, en provoquant une fermeture progressive de l'ouverture, dans lequel la résistance au flux d'air de l'atmosphère vers le canal d'échantillonnage augmente progressivement avec une diminution de la pression d'air à la sortie (12) de la sonde.
